# EUROPÄISCHE PATENTSCHRIFT

(11) **EP 1 432 409 B1**
(45) Veröffentlichungstag und Bekanntmachung des Hinweises auf die Patenterteilung: **17.09.2008**
(21) Anmeldenummer: 03790791.2
(22) Anmeldetag: 08.07.2003
(51) Int. Cl.: A61K 9/28, A61K 9/50, A61K 9/16

(54) **GRANULAT ODER PULVER ZUR HERSTELLUNG VON ÜBERZUGS- UND BINDEMITTELN FÜR ARZNEIFORMEN**
GRANULATE OR POWDER FOR PRODUCING COATING OR BINDING AGENTS FOR MEDICAMENTS
GRANULES OU POUDRE POUR LA PREPARATION DE COUVERTURE OU DE LIANT POUR DES FORMES MEDICAMENTEUSES

(30) Priorität: 27.08.2002 DE 10239999
(43) Veröffentlichungstag der Anmeldung: 30.06.2004
(73) Patentinhaber: Evonik Röhm GmbH, 64293 Darmstadt (DE)
(72) Erfinder: PETEREIT, Hans-Ulrich, 64291 Darmstadt (DE); MEIER, Christian, 64295 Darmstadt (DE); ROTH, Erna, 64297 Darmstadt (DE); GRYCZKE, Andreas, 64347 Griesheim (DE)
(86) Internationale Anmeldenummer: PCT/EP2003/007319
(87) Internationale Veröffentlichungsnummer: WO 2004/019918

(56) Entgegenhaltungen:
- EP-A- 0 164 669
- EP-A- 0 403 959
- EP-A- 0 727 205
- WO-A-00/05307
- WO-A1-02/06790

## Beschreibung

Die Erfindung betrifft ein Pulver oder Granulat zur Herstellung von Überzugs- und Bindemitteln für Arzneiformen.

### Stand der Technik

US 4 705 695 beschreibt ein Verfahren zum Überziehen von pharmazeutischen Formulierungen mit einem wäßrigen Überzugsmittel enthaltend ein wasserlösliches (Meth)acrylat-Copolymer mit tertiären Aminogruppen sowie ein wasserunlösliches, neutrales Polymer als Binder. Die Löslichkeit des (Meth)acrylat-Copolymers bestehend z. B. aus gleichen Anteilen Methylmethacrylat und Dimethylaminoethylmethacrylat, wird durch Einrühren in Pulverform mit Partikelgrößen unter 0,25 mm in Wasser unter gleichzeitiger Zugabe einer Säure bewirkt. Als Binder wird ein unlösliches Copolymer, z. B. aus Methylmethacrylat und Ethylacrylat (70 : 30), eingesetzt. Die Herstellung der Überzugslösung ist relativ aufwendig. Wegen des Gehaltes an Säure hat der Überzug einen unangenehmen Geschmack. Entsprechende Filme lösen sich sowohl in künstlichem Magensaft als auch in Wasser in weniger als zwei Minuten.

WO 00/05307 beschreibt ein Verfahren zur Herstellung eines Überzugs- und Bindemittels für orale oder dermale Arzneiformen bestehend aus (a) 35 - 98 Gew.-% eines Copolymers, bestehend aus radikalisch polymerisierten C1- bis C4-Estern der Acryl- oder Methacrylsäure und weiteren (Meth)acrylatMonomeren, die funktionelle tertiäre Aminogruppen aufweisen und (b) 1-50 Gew.-% eines Weichmachers sowie 1-15 Gew.-%, eines Emulgators mit einem HLB-Wert von mindestens 14 wobei die Komponenten (a), (b) und (c) mit oder ohne Zusatz von Wasser und gegebenenfalls unter Zusatz eines pharmazeutischen Wirkstoffs und weiterer üblicher Zuschlagstoffe miteinander vermengt werden und das Überzugs- und Bindemittel durch Schmelzen, Gießen, Ausstreichen oder Aufsprühen hergestellt wird, wobei das Copolymer (a) in Pulverform mit einer mittleren Teilchengröße von 1 - 40 µm eingebracht wird.

Ein Copolymer ensprechend der WO 00/05307 aus Methylmethacrylat, Butylmethacrylat, und Dimethylaminoethylmethacrylat in Verhältnis 25 : 25 : 50 mit einer mittleren Teilchengröße von 15 µm ist unter dem Namen EUDRAGIT® E PO (Eingetragene Marke der Röhm GmbH & Co. KG) im Handel und somit Stand der Technik.

Die Formulierung in der definierten Pulverform in Kombination mit Weichmacher und Emulgator ermöglicht es, die entsprechenden Copolymere ohne den Zusatz von Säuren in stabile wäßrige Lösungen bzw. Dispersionen zu überführen. Es besteht der Vorteil, daß ein ansonsten auftretender bitterer Eigengeschmack des Überzugsmittels vermieden werden kann. Die Überzugs- und Bindemittel sind zudem in Wasser kaum löslich, lösen sich aber in künstlichem Magensaft rasch auf. Sie eigenen sich daher insbesondere für geschmacksisolierende, im Magensaft rasch zerfallende Formulierungen.

Eine Weiterentwicklung der WO 00/05307 ist durch die Veröffentlichungen von Roth et al. ("Improved Moisture Protection with New Aqueous Aminomethacrylate Copolymer Formulations", CRS, Annual Meeting 2002 in Seoul (Korea) Juli 2002) und Petereit "new aspects of moisture protection and insulation of solid dosage forms with EUDRAGIT® EPO" 33th International EUDRAGIT^{®} Workshop 2001 (Darmstadt, 27-28 September 2001) bekannt geworden.

Es wird berichtet, daß der Zusatz von C12 - C18-Fettsäuren oder -Alkoholen einen Feuchtschutz bewirkt. Speziell wird der Zusatz von z. B. 10 Gew.-% Natrium-Laurylsulfat und 15 Gew.-% Stearinsäure zu EUDRAGIT® EPO in dispergierter Form empfohlen.

### Aufgabe und Lösung

Die Erfindung geht im wesentlichen von den eingangs genannten EUDRAGIT® EPO-Formulierungen mit Zusatz von C12 - C18-Fettsäuren oder -Alkoholen und Emulgatoren wie z. B Natrium-Laurylsulfat aus. Daraus hergestellte Überzugs- und Bindemittel weisen vorteilhaft eine nur geringe Wasserdampfdurchlässigkeit auf (Feuchtschutzwirkung). Die Publikationen von Roth et al. und Petereit empfehlen die Dispergierung der als Pulver vorliegenden Einzelkomponenten unter Rührbedingungen. Die Verwendung der pulverförmigen Komponenten hat den Nachteil, daß damit stets eine unerwünschte Bildung von Pulverstäuben einhergeht, was beim Endanwender in der Regel unerwünscht ist. Es wurde daher als Aufgabe gesehen, eine Zwischenstufe bereitzustellen, die sich vom Endanwender leicht und unter deutlich reduzierter Staubentwicklung zu einem Überzugs- und Bindemittel verarbeiten läßt.

Die Aufgabe wird gelöst durch ein

Verfahren zur Herstellung eines Granulats oder Pulvers, geeignet als Überzugs- und Bindemittel für orale oder dermale Arzneiformen, für Kosmetika oder Nahrungsergänzungsmittel, bestehend im wesentlichen aus
(a) einem Copolymer, bestehend aus radikalisch polymerisierten C1-bis C4-Estern der Acryl- oder Methacrylsäure und weiteren (Meth)acrylat-Monomeren die funktionelle tertiäre Aminogruppen aufweisen,
(b) 3 bis 25 Gew.-%, bezogen auf (a), eines Emulgators mit einem HLB-Wert von mindestens 14,
(c) 5 bis 50 Gew.-%, bezogen auf (a), einer C₁₂- bis C₁₈-Monocarbonsäure oder einer C₁₂- bis C₁₈-Hydroxylverbindung die ein Alkanol mit einer endständigen Hydroxylgruppe ist,
wobei die Komponenten (a), (b) und (c) gegebenenfalls unter Zusatz eines pharmazeutischen Wirkstoffs und/oder weiterer üblicher Zuschlagstoffe gleichzeitig oder nacheinander miteinander vermengt oder vermischt werden, in einem heizbaren Mischer geschmolzen, gemischt, die Schmelze abgekühlt und zu einem Granulat oder Pulver zerkleinert wird, wobei die Komponente (a) in Pulverform mit einer mittleren Teilchengröße von über 40 µm oder als Granulat mit einer mittleren Teilchengröße von 0,5-5 mm eingesetzt wird.

Durch den Extrusionsvorgang der Komponenten (a), (b) und (c) sowie gegebenenfalls zusätzlich zugegebener pharmazeutischer Wirkstoffe und/oder weiterer üblicher Zuschlagstoffe, entsteht offenbar eine molekulare Matrix, die nach dem Zerkleinern weitgehend staubfrei verarbeitbare Pulver oder Granulate ergibt. Der Endverbraucher kann die Granulate oder Pulver leicht in üblicher Weise weiterverarbeiten. Die Pulver oder Granulate ersparen das Vorhalten, Einwiegen und die Einarbeitung vieler Einzelkomponenten, da diese bereits in den vorgegebenen Mengen enthalten sind. Die Staubentwicklung unter Verwendung der erfindungsgemäßen Pulver oder Granulate wird vermieden. Überraschend ist es, daß sich auch Copolymere gemäß Komponente (a), die in Pulverform mit einer mittleren Teilchengröße von über 40 µm vorliegen, und die in dieser Form in der Regel längerer Dispergierzeiten, von z. B. mehreren Stunden, erfordern, auf diese Weise in eine rasch dispergierbare Form überführen lassen. Noch überraschender jedoch ist, dass sich mit der vorliegenden Erfindung sogar Granulate z. B. mit Teilchengrößen von 0,5 - 5 mm in eine rasch dispergierbare Form überführen lassen.

### Ausführung der Erfindung

### Komponente (a)

Die Copolymere (a) bestehen im wesentlichen oder ganz aus radikalisch polymerisierten C1- bis C4-Estern der Acryl- oder Methacrylsäure und weiteren (Meth)acrylat-Monomeren, die funktionelle tertiäre Aminogruppen, aufweisen.

Geeignete Monomere mit funktionellen tertiäre Aminogruppen sind in US 4 705 695, Spalte 3, Zeile 64 bis Spalte 4, Zeile 13 aufgeführt. Insbesondere zu nennen sind Dimethylaminoethylacrylat, 2-Dimethylaminopropylacrylat, Dimethylaminopropylmethacrylat, Dimethylaminobenzylacrylat, Dimethylaminobenzylmethacrylat, (3-Dimethylamino-2,2-dimethly)propylacrylat, Dimethylamino-2,2-dimethly)propylmethacrylat, (3-Diethylamino-2,2-dimethly)propylacrylat und Diethylamino-2,2-dimethly)propylmethacrylat. Besonders bevorzugt ist Dimethylaminoethylmethacrylat.

Der Gehalt der Monomere mit tertiären Aminogruppen im Copolymeren kann vorteilhafterweise zwischen 30 und 70 Gew.-%, bevorzugt zwischen 40 und 60 Gew.-% liegen. Der Anteile der C1- bis C4-Estern der Acryl- oder Methacrylsäure beträgt 70 - 30 Gew.-%. Zu nennen sind Methylmethacrylat, Ethylmethacrylat, Butylmethacrylat, Methylacrylat, Ethylacrylat, Butylacrylat.

Ein der Komponente (a) entsprechendes (Meth)acrylatcopolymer mit tertiären Aminogruppen kann z. B. aus 20 - 30 Gew.-% Methylmethacrylat, 20 - 30 Gew.-% Butylmethacrylat und 60 - 40 Gew.-% Dimethylaminoethylmethacrylat aufgebaut sein. Der Anteil der Komponente (a) an der Formulierung beträgt bevorzugt 50 - 90 Gew.-%.

Die Copolymere (a) werden in an sich bekannter Weise durch radikalische Substanz-, Lösungs-, Perl- oder Emulsionspolymerisation erhalten. Sie werden vor der Verarbeitung durch geeignete Mahl-, Trocken- oder Sprühprozesse in eine mit den Komponenten (b) und (c) trocken bzw. in der Schmelze mischbare Form gebracht. Insbesondere ist die Granulat- oder Pulverform zur Weiterverarbeitung geeignet. Geeignete Gerätschaften dafür sind dem Fachmann geläufig, z. B. Heißabschlaggranulatoren, Stranggranulatoren, Luftstrahlmühlen, Stiftmühlen, Fächermühlen. Gegebenenfalls können entsprechende Siebungsschritte einbezogen werden. Eine geeignete Mühle für industrielle Großmengen zur Herstellung von Pulvern ist zum Beispiel eine Gegenstrahlmühle (Multi Nr. 4200), die mit ca. 6 bar Überdruck betrieben wird. Geeignete Pulver können z. B. mittlere Teilchengrößen von 1 - 40 µm (EUDRAGIT® EPO-Typ) oder zwischen 40 µm bis 500 µm. Granulate können z. B. abgerundet linsenförmig oder zylinderförmig sein mit Teilchengrößen von 0,5 bis 50 mm, bevorzugt 1 bis 5 mm (EUDRAGIT® E-Typ).

### Komponente (b)

Emulgatoren oder Tenside sind grenzflächenaktive Substanzen mit lyobipolarem Charakter, d.h. in ihrem Molekül müssen unpolare, lipophile und polare, hydrophile Zentren vorliegen (P.H. List, Arzneiformenlehre, Wissenschaftliche Verlagsgesellschaft-mbH Stuttgart,1982,Kap. 6.2.). Je nach molekularem Aufbau unterscheidet man zwischen ionogenen und nichtionogen Emulgatoren.

Der HLB-Wert ist ein 1950 von Griffin eingeführtes Maß der Hydrophilie bzw. Lipophilie von nichtionischen Tensiden. Er läßt sich experimentell durch die Phenol-Titrationsmethode nach Marszall bestimmen; vgl. "Parfümerie, Kosmetik", Band 60, 1979, S. 444 - 448; weitere Literaturhinweise in Römpp, Chemie-Lexikon, 8.Aufl. 1983, S.1750. Siehe weiterhin z. B. US 4 795 643 (Seth)).

Ein HLB-Wert (Hydrophile/Lipophile Balance) läßt sich nur bei nicht ionischen Emulgatoren exakt bestimmen. Bei anionischen Emulgatoren kann dieser Wert rechnerisch ermittelt werden, liegt jedoch praktisch immer über oder weit über 14.

Unter Emulgatoren (b) mit einem HLB-Wert über 14 werden erfindungsgemäß hydrophile, nicht ionische Emulgatoren mit HLB - Bereich von mindestens 14 sowie ebenfalls hydrophile, anionische Emulgatoren und deren Salze, die einen rechnerischen HLB-Wert über 14 aufweisen, verstanden. Emulgatoren mit HLB-Werten von weniger als 14, wie z. B. Glycerolmonostearat können zwar zusätzlich ebenfalls enthalten sein, ersetzen jedoch die Emulgatoren (b) mit HLB-Werten von mindestens 14 nicht.

Geeignete Emulgatoren (b) sind z. B. Natriumlaurylsulfat und Natriumcetylstearylsulfat, Saccharosestearat und Polysorbat 80. Die Emulatoren (b) sind in Mengen von 1 - 25, bevorzugt 5 - 10 Gew.-% bezogen auf die Komponente (a) enthalten. Möglich ist natürlich auch der Einsatz von Emulgatormischungen.

Die Zugabe der Emulgatoren (b) zur Formulierung kann in bekannter Weise, direkt, in wäßriger Lösung oder nach thermischer Vorbehandlung der Mischung vorgenommen werden.
Die Emulgatoren können je nach Typ (lipophil oder hydrophil) und zugesetzter Menge die Funktionalität der Polymerschicht beeinflussen.

### Komponente (c)

Komponente (c): 5 bis 50, bevorzugt 10 bis 20 Gew.-% (bezogen auf die Komponente (a)) einer C₁₂- bis C₁₈-Monocarbonsäure oder einer C₁₂- bis C₁₈-Hydroxylverbindung die ein Alkanol mit einer endständigen Hydroxylgruppe ist. Die Komponente (c) ist entscheidend für die überraschend geringe Wasserdampfdurchlässigkeit der Formulierungen.
Bevorzugt sind unverzweigte C₁₂- bis C₁₈-Monocarbonsäure oder einer C₁₂- bis C₁₈-Hydroxylverbindungen. Es können gegebenenfalls auch verzweigte Derivate der genannten Substanzen geeignet sein.

C₁₂- bis C₁₈-Monocarbonsäuren sind z.B insbesondere Laurinsäure und Myristinsäure. Bevorzugt sind Palmitinsäure und Stearinsäure.

C₁₂- bis C₁₈-Hydroxylverbindung, ein Alkanol mit einer endständigen Hydroxylgruppe wie z. B. Laurylalkohol oder Stearylalkohol.

### Weitere Zuschlagstoffe

Der erfindungsgemäßen Formulierung werden bevorzugt bei der Herstellung der Granulate oder Pulver übliche Zuschlagstoffe hinzugefügt. Die Zuschlagstoffe können auch noch bei der Verarbeitung zum Überzugs- und Bindemittel hinzugefügt werden. Grundsätzlich müssen natürlich alle eingesetzten Substanzen toxikologisch unbedenklich und insbesondere in Arzneimitteln ohne Risiko für Patienten zu verwenden sein.

Einsatzmengen und Verwendung der üblichen Zuschlagstoffe in Arzneimittelüberzügen oder Beschichtungen sind dem Fachmann geläufig. Übliche Zuschlagstoffe können z. B. Trennmittel, Pigmente, Stabilisatoren, Antioxidantien, Porenbildner, Penetrationsförderer, Glanzmittel, Aromastoffe oder Geschmacksmittel sein. Sie dienen als Verarbeitungshilfsmittel und sollen ein sicheres und reproduzierbares Herstellungsverfahren sowie gute Langzeitlagerstabilität gewährleisten oder sie erreichen in der Arzneiform zusätzliche vorteilhafte Eigenschaften. Sie werden den Polymerzubereitungen vor der Verarbeitung zugesetzt und können die Permeabilität der Überzüge beeinflussen, was ggf. als zusätzlicher Steuerparameter genutzt werden kann.

### • Trennmittel:

Trennmittel besitzen in der Regel lipophile Eigenschaften und werden in der Regel den Sprühsuspensionen zugesetzt. Sie verhindern eine Agglomeration der Kerne während der Befilmung. Bevorzugt werden Talkum, Mg- oder Ca - Stearat, gemahlene Kieselsäure, Kaolin oder nicht ionische Emulgatoren mit einem HLB - Wert zwischen 3 und 8 eingesetzt. Übliche Einsatzmengen für Trennmittel in den erfindungsgemäßen Überzugs- und Bindemitteln liegen zwischen 0,5 bis 100 Gew.-% bezogen auf das Copolymer (a).

### • Pigmente:

Mit dem Überzugsmittel unverträgliche Pigmente sind insbesondere solche Pigmente, die wenn sie der (Meth)acrylat-Copolymer-Dispersion direkt zugesetzt werden, z. B. durch Einrühren, in üblichen Anwendungsmengen von z. B. 20 bis 400 Gew.-% bezogen auf das Trockengewicht des (Meth)acrylat-Copolymeren zur Destabilisierung der Dispersion, Koagulation, zu Entmischungserscheinungen oder ähnlich unerwünschten Effekten führen. Weiterhin sind die zu verwendenden Pigmente natürlich nicht toxisch und für pharmazeutische Zwecke geeignet. Siehe dazu z. B. auch: Deutsche Forschungsgemeinschaft, Farbsfoffe für Lebensmittel, Harald Boldt Verlag KG, Boppard (1978); Deutsche Lebensmittelrundschau 74, Nr. 4, S. 156 (1978); Arzneimittelfarbstoffverordnung AmFarbV vom 25.08.1980.

Mit dem Überzugsmittel unverträgliche Pigmente können z. B. Aluminiumoxidpigmente sein. Unverträgliche Pigmente sind z. B., Gelborange , Cochenillerotlack, Farbpigmente auf Basis von Aluminiumoxid bzw Azofarbstoffen, Sulfonsäurefarbstoffe, Gelborange S (E110, C.I. 15985, FD&C Yellow 6), Indigocarmin (E132, C.I. 73015, FD&C Blue 2), Tartrazin (E 102, C.I. 19140, FD&C Yellow 5), Ponceau 4R (E 125, C.I. 16255, FD&C Cochineal Red A), Chinolingleb (E 104, C.I. 47005, FD&C Yellow 10), Erythrosin (E127, C.I. 45430, FD&C Red 3), Azorubin (E 122, C.I. 14720, FD&C Carmoisine), Amaranth (E 123, C. I. 16185, FD&C Red 2), Brilliantsäuregrün (E 142, C.I. 44090, FD&C Green S).

Die angegebenen E-Nummern der Pigmente beziehen sich auf eine EU-Nummerierung. Siehe dazu auch "Deutsche Forschungsgemeinschaft, Farbstoffe für Lebensmittel, Harald Boldt Verlag KG, Boppard (1978); Deutsche Lebensmittelrundschau 74, Nr. 4, S. 156 (1978); Arzneimittelfarbstoffverordnung AmFarbV vom 25.08.1980. Die FD&C-Nummem beziehen sich auf die Zulassung in Food, Drugs und Cosmetics durch U.S. Food and Drug Administration (FDA) beschrieben in: U.S. Food and Drug Administration, Center for Food Safety and Applied Nutrition, Office of Cosmetics and Colors: Code of Federal Regulations - Title 21 Color Additive Regulations Part 82, Listing of Certified Provisionally Listed Colors and Specifications (CFR 21 Part 82).

### • Weichmacher

Weitere Zuschlagstoffe können auch Weichmacher sein. Übliche Mengen liegen zwischen 0 und 50, bevorzugt 0 bis 20, insbesondere 0 bis 10 Gew.-%. Besonders bevorzugt sind allerdings höchstens 5 Gew.-% oder kein Weichmacher enthalten, da die Formulierungen durch die Anwesenheit der Komponenten (c) häufig bereits elastisch genug sind und zusätzlicher Weichmacher zu unerwünschter Klebrigkeit führen kann.

Weichmacher können je nach Typ (lipophil oder hydrophil) und zugesetzter Menge die Funktionalität der Polymerschicht beeinflussen. Weichmacher erreichen durch physikalische Wechselwirkung mit dem Polymeren eine Absenkung der Glasübergangstemperatur und fördern in Abhängigkeit von der zugesetzten Menge die Verfilmung. Geeignete Stoffe haben in der Regel ein Molekulargewicht zwischen 100 und 20.000 und enthalten eine oder mehrere hydrophile Gruppen im Molekül, z. B. Hydroxyl-, Ester- oder Aminogruppen.

Beispiele geeigneter Weichmacher sind Citronensäurealkylester, Glycerinester, Phthalsäurealkylester, Sebacinsäurealkylester, Succroseester, Sorbitanester, Diethylsebacat, Dibutylsebacat und Polyethylenglykole 200 bis 12.000. Bevorzugte Weichmacher sind Triethylcitrat (TEC), Acetyltriethylcitrat (ATEC) und Dibutylsebacat (DBS). Weiterhin zu nennen sind in der Regel bei Raumtemperatur flüssige Ester wie Citrate, Phthalate, Sebacate oder Rizinusöl. Bevorzugt werden Zitronensäure- und Sebacinsäureester verwendet.

Die Zugabe der Weichmacher zur Formulierung kann in bekannter Weise, direkt, in wäßriger Lösung oder nach thermische Vorbehandlung der Mischung vorgenommen werden. Auch können Mischungen von Weichmachern eingesetzt werden.

### Verarbeitung zu Granulaten oder Pulvern

Die Komponenten (a), (b) und (c) werden gegebenenfalls unter Zusatz eines pharmazeutischen Wirkstoffs und/oder weiterer üblicher Zuschlagstoffe gleichzeitig oder nacheinander miteinander vermengt oder vermischt.
Die Komponenten werden anschließend in einem heizbaren Mischer geschmolzen, gemischt, die Schmelze wird abgekühlt und zu einem Granulat oder Pulver zerkleinert.

Man kann die Komponenten (a), (b) und (c) sowie den gegebenenfalls vorhandenen pharmazeutischen Wirkstoff und/oder weitere übliche Zuschlagstoffe z. B. trocken, z. B. in einem Freifallmischer oder einem Zwangsmischer miteinander vermischen. Man kann jedoch auch die Komponenten (a), (b) und (c) sowie den gegebenenfalls vorhandenen pharmazeutischen Wirkstoff und/oder weitere übliche Zuschlagstoffe alleine oder als Teilmischung über kontinuierlich arbeitende Dosierautomaten direkt einen heizbaren Mischer, z. B. einen Extruder, zuführen. Man kann auch die Komponenten (b) und (c) sowie den gegebenenfalls vorhandenen pharmazeutischen Wirkstoff und/oder weitere übliche Zuschlagstoffe alleine oder als Teilmischung trocken oder gegebenenfalls im Schmelzezustand in die Schmelze der Komponente (a) dosieren.

Die Mischung wird bevorzugt in einem Extruder geschmolzen und mittels eines Extruders extrudiert, z. B. bei Temperaturen im Bereich von 80 bis 160, bevorzugt 100 bis 150 °C extrudiert. Es kann sich um einen Einschnecken- oder einen Doppelschneckenextruder handeln, an dessen Ende das Extrudat z. B. als Schmelzestrang auftritt. Das erhaltene Extrudat wird zu einem Granulat oder Pulver zerkleinert. Dies kann z. B. durch einen Heißabschlaggranulator, einem Stranggranulator und/oder Mahlen in einer Mühle, z. B. einer Stiftmühle erfolgen. Der Strang kann z. B. wird über ein Abzugsband abgezogen und einem Stranggranulator zugeführt werden, welcher den Strang in 1 bis 5 mm lange Stücke zerschneidet. Bei Granulaten kann die mittlere Korngröße z. B. im Bereich von 0,5 bis 5 mm, bevorzugt 1 bis 3 mm liegen. Geeignete Pulvergrößen können im Bereich von 1 bis 500 µm bevorzugt 100 bis 500 µm liegen.

### Das Weiterverarbeitung der erfindungsgemäßen Pulver oder Granulate

Die Erfindung betrifft demnach Granulate oder Pulver , die mittels Extrusion und anschließendes Zerkleinern des Extrudats herstellbar sind und sich eignen als eine in Wasser leicht dispergierbare oder schmelzbare Vorstufe, bzw. als Zwischenprodukt, für ein Überzugs- und Bindemittel für orale oder dermale Arzneiformen.

Die Granulate oder Pulver können bei Raum- oder erhöhter Temperatur mit oder ohne Zusatz von Wasser und gegebenenfalls unter Zusatz noch nicht enthaltener pharmazeutischen Wirkstoffe und/oder weiterer üblicher Zuschlagstoffe in an sich bekannter Weise durch Schmelzen, Gießen, Ausstreichen, Aufsprühen oder Granulieren zu Überzugs- und Bindemittel weiterverarbeitet werden. Dabei ist die Verfilmung des Überzugs- und Bindemittels Voraussetzung für den funktionellen Effekt in den resultierenden Arzneiformen.

Bevorzugt ist das Überführen der Granulate oder Pulver in eine Dispersion durch Eintragen in Wasser und Rühren bei Raumtemperatur. Alternativ können höhere Scherkräfte oder erhöhte Temperaturen, z.B. 50 bis 70 °C angewendet werden. Unter diesen Bedingungen kann die Dispergierzeit in etwa 45 bis 120 min betragen.

Der Feststoffgehalt der entstandenen Dispersion liegt zwischen 5 und 30 Gew.-%, bevorzugt zwischen 10 und 20 Gew.-%). Die Viskosität der Dispersion hängt von der Konzentration des Feststoffs ab. Sie liegt in der Regel unter 1 Pa*s.

Zweier oder mehrere Granulate oder Pulver, die jeweils verschiedene Farbpigment enthalten, können durch Mischung zu einem Überzugs- und Bindemittel verarbeitet werden. Dies hat den Vorteil, daß man Farbmischungen erzeugen kann. Man auf diese Weise Farbtöne erzeugen, die mit einzelnen Farbpigmenten nicht darstellbar sind.

Die Verfilmung erfolgt, unabhängig von dem Auftragsverfahren durch Zuführung von Energie. Dies kann über Konvektion (Wärme), Strahlung (Infrarot oder Mikrowellen) oder Leitung erfolgen. Für den Auftrag als Suspensionsmittel eingesetztes Wasser verdampft dabei, gegebenenfalls kann auch ein Vakuum angewendet werden, um das Verdampfen zu beschleunigen. Die für die Verfilmung notwendige Temperatur hängt von der Kombination der eingesetzten Komponenten ab.

### Weiterverarbeitung der erfindungsgemäßen Pulver oder Granulate zur Herstellung von Bindemitteln:

Die Verwendung als Bindemittel erfolgt z. B. durch Aufsprühen der wäßrigen Polymersuspension auf wirkstofffreie Kerne (Nonpareilles) bei gleichzeitiger Zugabe von pulverförmigem Wirkstoffen oder deren Mischungen.
Eine weitere Ausführungsform ist das Aufsprühen der wäßrigen Polymersuspension gemeinsam mit darin gelösten oder suspendierten Wirkstoffen.

Die Granulate oder Pulver können auch durch Verarbeitung mittels Feucht- oder Schmelzgranulation als Bindemittel weiterverarbeitet werden.

### Weiterverarbeitung der erfindungsgemäßen Formulierung zur Herstellung von Überzugsmittel:

Die Granulate oder Pulver können als feuchtigkeitsisolierende und/oder als geschmacksisolierender Überzugsmittel verwendet werden.

Vorgeformte Träger für die Überzüge sind Kapseln, Tabletten, Granulate, Pellets, Kristalle von regelmäßiger oder unregelmäßiger Form. Die Größe von Granulaten, Pellets oder Kristallen liegt zwischen 0,01 und 2,5 mm, die von Tabletten zwischen 2,5 und 30,0 mm. Kapsel bestehen aus Gelatine, Stärke oder Cellulosederivaten.

Pulver und Kristalle enthalten in der Regel 100 % der biologisch aktiven Substanz. Vorgeformte Träger enthalten von etwa 0,1 zu bis 99 % der biologisch aktiven Substanz bzw. des pharmazeutischen Wirkstoffs, sowie bis 1 bis 99,9 Gew.-%.weitere pharmazeutische Hilfsstoffe.

Übliche Herstellungsverfahren sind direktes Verpressen, Verpressen von Trocken-, Feucht- oder Sintergranulaten, Extrusion und anschließende Ausrundung, feuchte oder trockene Granulation oder direkte Pelletierung (z.B. auf Tellern) oder durch Binden von Pulvern (Powder layering) auf wirkstofffreie Kugeln (Nonpareilles) oder wirkstoffhaltige Partikeln.

### Neben dem Wirkstoff können sie weitere pharmazeutische Hilfsstoffe enthalten:

Bindemittel, wie Zellulose und deren Derivate, Polyvinylpyrrolidon (PVP), Feuchthaltemittel, Zerfallsförderer, Gleitmittel, Sprengmittel, (Meth)acrylate, Stärke und deren Derivate, Zucker Solubilisatoren oder andere.

Von besonderer Bedeutung ist die Zerfallszeit der Kerne, die die Freigabe des Wirkstoffs beeinflußt. Man strebt heute kurze Zerfallszeiten von unter 5, bzw. unter 10 min im Zerfallstest nach Ph. Eur. an. Längere Zerfallszeiten sind deswegen problematisch, weil zusätzliche Überzüge die Freigabe des Wirkstoffs weiter verzögern und den therapeutischen Effekt in Frage stellen können. Als Grenzwert wird heute eine Zerfallszeit von 30 min angesehen. Man testet in Wasser und künstlichem Magensaft (0,1 N HCl).
Die eingesetzten Kerne sind homogen oder haben einen schichtartigen Aufbau. Sind Gravuren in die Oberflächen eingelassen, sollen diese durch Überzüge möglichst überdeckt aber wenig ausgefüllt werden. Die erfindungsgemäß eingesetzte Schichtdicke des Polymerpulvers variiert stark und hängt von dem Verarbeitungsverfahren oder der Menge an Zuschlagstoffen ab. Sie liegt zwischen 1 und 100 µm, bevorzugt zwischen 10 und 50 µm. Auf üblichen Tabletten entspricht das einem Polymerauftrag von 0,5 bis 5 Gew.-%.

Überzogene Mikropartikel können gemäß K. Lehmann et al., Drugs made in Germany 37, 2, 53-60 (1994) und T.E. Beckert et al, International Journal of Pharmaceutics 143,(1996), 13-23 zu zerfallenden Tabletten verpreßt werden, ohne signifikanten Einfluss auf die Funktion des Polymers.

Die Funktion der verfilmten Polymerschicht in der endgültigen Arzneiform kann vielfältig sein:
- Schutz vor schädlichen Umwelteinflüssen durch Feuchte, Gase, Licht usw.
- Geruchs- oder Geschmacksisolierung,
- Kennzeichnung durch Farbe
- Mechanische Stabilisierung
- Isolierung unverträglicher Inhaltsstoffe
- Vermeidung von Haftung an den Schleimhäuten.
- Zeitlich verzögerte Wirkstoffabgabe
- pH - gesteuerte Wirkstoffabgabe
- Isolierung von Kernen gegenüber weiteren Überzügen

Vorteilhaft ist die niedrige Viskosität der Polymermischung in wäßriger Dispersion auch bei hohen Feststoffanteilen bis zu 30 %, da Gravuren auf der Oberfläche von Tabletten detailliert nachgebildet werden.
Besonders vorteilhaft ist die gute Schutz- und Isolierwirkung der erfindungsgemäßen Polymermischung bei gleichzeitig geringem Einfluß auf den Tablettenzerfall. Schon bei geringen Polymeraufträgen von 1 Gew.-% schon eine Geschmacksisolierung von mehr als 30 sec. erreicht. Dickere Überzüge mit einem Copolymer aus Methylmethacrylat, Butylmethacrylat, und Dimethylaminoethylmethacrylat in Verhältnis 25 : 25 : 50 (EUDRAGIT^{®} E oder EUDRAGIT^{®} EPO) verbessern die Geschmacksabdeckung, ohne jedoch die Zerfallszeit in 0, 1 N HCl zu verlängern. Ebenso vorteilhaft ist die zuverlässige Abdeckung gefärbter Kerne durch Überzüge mit hohem Pigmentanteil. Eine besondere Ausführungsform ist die Einbettung eines zweiten Wirkstoffs in den Überzug auf einen wirkstoffhaltigen Kern.

### Auftrag auf der erfindungsgemäßen Formulierung zur Herstellung auf Träger

Die erfindungsgemäße Formulierung kann in Pulverform, als Schmelze oder in wäßriger Suspension durch Granulieren, Gießen, Ausstreichen oder mittels Sprühauftrag angewendet werden. Wasser dient dabei hauptsächlich als Vehikel, um dünne Umhüllungen gleichmäßig auf sphärische Kerne z. B. durch Sprühen aufzutragen. Für Beschichtungen werden außerdem Streichverfahren eingesetzt. Das eingesetzte Verfahren richtet sich hauptsächlich nach dem gewählten Träger. Trockene Pulver werden durch Ausstreichen oder Bestäuben aufgetragen, ggf. auch unter Einsatz elektrostatische Kräfte. Die Verfilmung kann durch Einwirkung von Wärme erfolgen. Für die Ausführung ist dabei entscheidend; dass gleichmäßige, geschlossene Schichten entstehen.

Auftragsverfahren gemäß dem Stand der Technik s. z. B. Bauer, Lehmann, Osterwald, Rothgang, "Überzogene Arzneiformen" Wissenschaftliche Verlagsgesellschaft mbH Stuttgart, Kap 7, S.165 -196
Für die Applikation relevante Eigenschaften, geforderte Tests und Spezifikationen sind in Arzneibüchem aufgelistet.
Details sind den gängigen Lehrbüchern zu entnehmen, z. B.:
- Voigt, R. (1984): Lehrbuch der pharmazeutischen Technologie; Verlag Chemie Weinheim - Beerfield Beach/Florida - Basel.
- Sucker, H., Fuchs, P., Speiser, P.: Pharmazeutische Technologie, Georg Thieme Verlag Stuttgart (1991), insbesondere Kapitel 15 und 16, S. 626 - 642.
- Gennaro, A.,R. (Editor), Remington's Pharmaceutical Sciences, Mack Publishing Co., Easton Pennsylvania (1985), Chapter 88, S. 1567 - 1573.
- List, P. H. (1982): Arzneiformenlehre, Wissenschaftliche Verlagsgesellschaft mbH, Stuttgart.

### Wasserdampfdurchlässigkeit

Während Überzugs- und Bindemittel nach WO 00/05307 Wasserdampfdurchlässigkeiten gemessen nach DIN 53122 im Bereich von 400 (g/m²/d) oder darüber weisen, liegen die Wasserdampfdurchlässigkeiten der aus den erfindungsgemäßen Pulvern oder Granulaten hergestellten Überzugs- und Bindemittel bei von höchstens 350 (g/m²/d), bevorzugt höchtens 300 (g/m²/d), besonders bevorzugt höchstens 200 (g/m²/d). Insbesondere können die erfindungsgemäßen Pulver oder Granulate in vielen Fällen auch unter weitgehendem oder völligen Verzicht auf übliche Weichmacher weiterverarbeitet werden. Dies ist vorteilhaft, da man stets bemüht ist, die Zahl der Komponenten bei Arzneimittelformulierungen gering zu halten.

### Biologisch aktive Substanzen:

Die im Sinne der Erfindung eingesetzten Arzneistoffe sind dazu bestimmt, am oder im menschlichen oder tierischen Körper Anwendung zu finden, um
1. Krankheiten, Leiden, Körperschäden oder krankhafte Beschwerden zu heilen, zu lindern, zu verhüten oder zu erkennen.
2. die Beschaffenheit, den Zustand oder die Funktionen des Körpers oder seelische Zustände erkennen lassen.
3. vom menschlichen oder tierischen Körper erzeugte Wirkstoffe oder Körperflüssigkeiten zu ersetzen.
4. Krankheitserreger, Parasiten oder körperfremde Stoffe abzuwehren, zu beseitigen oder unschädlich zu machen oder
5. die Beschaffenheit, den Zustand oder die Funktionen des Körpers oder seelische Zustände zu beeinflussen.

Gebräuchliche Arzneistoffe sind Nachschlagewerken, wie z.B. der Roten Liste oder dem Merck Index zu entnehmen.
Die erfindungsgemäße Formulierung eignet sich zur Verabreichung grundsätzlich beliebiger pharmazeutischer Wirkstoffe, die vorzugsweise in isolierte oder geschützter Form verabreicht werden können, wie Antidepressiva, Betarezeptorenblocker, Antidiabetika, Analgetika, Antiphlogistika, Antirheumatika,Antihypotonika, Antihypertonika, Psychopharmaka, Tranquilizer, Antiemetika, Muskelrelaxantien, Glucocorticoide, Mittel zur Behandlung von Colitis ulcerosa oder Morbus Crohn, Antiallergika, Antibiotika, Antiepileptika, Antikoagulantia, Antimykotika, Antitussiva, Arteriosklerosemittel, Diuretika, Enzyme, Enzyminhibitoren, Gichtmittel, Hormone und deren Hemmstoffe, Herzglykoside, Imrnuntherapeutika und Zytokine, Laxantien, Lipidsenker, Magen-Darmtherapeutika, Migränemittel, Mineralstoffpraparate, Otologika, Parkinsonmittel, Schilddrüsentherapeutika, Spasmolytika, Thrombozytenaggregationshemmer, Vitamine, Zytostatika und Metastasenhemmer, Phytopharmaka, Chemotherapeutika und Aminosäuren.

Beispiele geeigneter Wirkstoffe sind Acarbose, , Nichtsteroidale Antirheumatia, Herzglykoside, Acetylsalicylsäure, Virustatika, Aclarubicin, Acyclovir, Cisplatin, Actinomycin, alpha- und beta-Sympatomimetika, (Allopurinol, Alosetron, Alprostadil, Prostaglandine, Amantadin, Ambroxol, Amlodipin, Methotrexat, S-Aminosa-licylsäure, Amitriptylin, Amlodipin, Amoxicillin, Anastrozol, Atenolol, Atorvastatin, Azathioprin, Balsalazid, Beclomethason, Betahistin, Bezafibrat, Bicalutamid, Diazepam und Diazepamderivate, Budesonid, Bufexamac, Buprenorphin, Methadon, Calciumsalze, Kaliumsalze, Magnesiumsalze, Candesartan, Carbamazepin, Captopril, Cefalosporine, Celetoxib, Cetirizin, Chenodeoxycholsäure, Ursodeoxycholsäure, Theophyllin und Theophyllinderivate, Trypsine, Cimetidin, Clarithromycin, Clavulansäure, Clindamycin, Clobutinol, Clonidin, Cotrimoxazol, Codein, Coffein, Vitamin D und Derivate von Vitamin D, Colestyramin, Cromoglicinsäure, Cumarin und Cumarinderivate, Cystein, Cytarabin, Cyclophosphamid, Ciclosporin, Cyproteron, Cytarabin, Dapiprazol, Desogestrel, Desonid, Dihydralazin, Diltiazem, Mutterkornalkaloide, Dimenhydrinat, Dimethylsulfoxid, Dimeticon, Dipyridarnoi, Domperidon und Domperidanderivate, Donepzil, Dopamin, Doxazosin, Doxorubizin, Doxylamin, Dapiprazol, Benzodiazepine, Diclofenac, Glykosidantibiotika, Desipramin, Econazol, ACE-Hemmer, Enalapril, Ephedrin, Epinephrin, Epoetin und Epoetinderivate, Morphinane, Calciumantagonisten, Irinotecan, Modafinil, Orlistat, Peptidantibiotika, Phenytoin, Riluzole, Risedronat, Sildenafil, Topiramat, Makrolidantibiotika, Esomeprazol, Estrogen und Estrogenderivate, Gestagen und Gestagenderivate, Testosteron und Testosteronderivate, Androgen und Androgenderivate, Ethenzamid, Etofenamat, Etofibrat, Fenofibrat, Etofyllin, Etoposid, Famciclovir, Famotidin, Felodipin, Fenofibrat, Fentanyl, Fenticonazol, Gyrasehemmer, Fluconazol, Fludarabin, Flunarizin, Fluorouracil, Fluoxetin, Flurbiprofen, Ibuprofen, Flutamid, Fluvastatin, Follitropin, Formoterol, Fosfomicin, Furosemid, Fusidinsäure, Galantamin, Gallopamil, Ganciclovir, Gemfibrozil, Gentamicin, Ginkgo, Johanniskraut, Glibenclamid, Harnstoffderivate als orale Antidiabetika, Glucagon, Glucosamin und Glucosaminderivate, Glutathion, Glycerol und Glycerolderivate, Hypothalamushormone, Goserelin, Gyrasehemmer, Guanethidin, Halofantrin, Haloperidot, Heparin und Heparinderivate, Hyaluronsäure, Hydralazin, Hydrochlorothiazid und Hydrochlorothiazidderivate, Salicylate, Hydroxyzin, Idarubicin, lfosfamid, Imipramin, Indometacin, Indoramin, Insulin, Interferone, Jod und Jodderivate, Isoconazol, Isoprenalin, Glucitol und Glucitolclerivate, Itraconazol, Ketoconazol, Ketoprofen, Ketotifen, Lacidipin, Lansoprazol, Levodopa, Levomethadon, Schilddrüsenhormone, Liponsäure und Liponsäurederivate, Lisinopril, Lisurid, Lofepramin, Lomustin, Loperamid, Loratadin, Maprotilin, Mebendazol, Mebeverin, Meclozin, Mefenaminsäure, Mefloquin, Meloxicam, Mepindolol, Meprobamat,Meropenem, Mesalazin, Mesuximid, Metamizol, Metformin, Methotrexat, Methylphenidat, Methylprednisolon, Metixen, Metoclopramid, Metoprolol, Metronidazol, Mianserin, Miconazol, Minocyclin, Minoxidil, Misoprostol, Mitomycin, Mizolastin, Moexipril, Morphin und Morphinderivate, Nachtkerze, Nalbuphin, Naloxon, Tilidin, Naproxen, Narcotin, Natamycin, Neostigmin, Nicergolin, Nicethamid, Nifedipin, Nifluminsäure, Nimodipin, Nimorazol, Nimustin, Nisoldipin, Adrenalin und Adrenalinderivate, Norfloxacin, Novaminsulfon, Noscapin, Nystatin, Ofloxacin, Olanzapin, Olsalazin, Omeprazol, Omoconazol, Ondansetron, Orlistat, Oseltamivir, Oxaceprol, Oxacillin, Oxiconazol, Oxymetazolin, Pantoprazol, Paracetamol, Paroxetin, Penciclovir, orale Penicilline, Pentazocin, Pentifyllin, Pentoxifyllin, Perphenazin, Pethidin, Pflanzenextrakte, Phenazon, Pheniramin, Barbitursäurederivate, Phenylbutazon, Phenytoin, Pimozid, Pindolol, Piperazin, Piracetam, Pirenzepin, Piribedil, Piroxicam, Pramipexol, Pravastatin, Prazosin, Procain, Promazin, Propiverin, Propranolol, Propyphenazon, Prostaglandine, Protionamid, Proxyphyllin, Quetiapin, Quinapril, Quinaprilat, Ramipril, Ranitidin, Reproterol, Reserpin, Ribavirin, Rifampicin, Risperidon, Ritonavir, Ropinirol, Rosiglitazon, Roxatidin, Roxithromycin, Ruscogenin, Rutosid und Rutosidderivate, Sabadilla, Salbutamol, Salmeterol, Scopolamin, Selegilin, Sertaconazol, Sertindol, Sertralion, Sildenafil, Silikate, Simvastatin, Sitosterin, Sotalol, Spagluminsäure, Sparfloxacin, Spectinomycin, Spiramycin, Spirapril, Spironolacton, Stavudin, Streptomycin, Sucralfat, Sufentanil, Sulbactam, Sulfonamide, Sulfasalazin, Sulpirid, Sultamicillin, Sultiam, Sumatriptan, Suxamethoniumchlorid, Tacrin, Tacrolimus, Tadalafil, Taliolol, Tamoxifen, Taurolidin, Tazaroten, Tegaserod, Temazepam, Teniposid, Tenoxicam, Terazosin, Terbinafin, Terbutalin, Terfenadin, Terlipressin, Tertatolol, Tetracycline, Tetryzolin, Theobromin, Theophyllin, Butizin, Thiamazol, Phenothiazine, Thiotepa, Tiagabin, Tiaprid, Propionsaurederivate, Ticlopidin, Timolol, Tinidazol, Tioconazol, Tioguanin, Tioxolon, Tiropramid, Tizanidin, Tolazolin, Tolbutamid, Tolcapon, Tolnaftat, Tolperison, Topotecan, Torasemid, Anti6strogene, Tramadol, Tramazolin, Trandolapril, Tranylcypromin, Trapidil, Trazodon, Triamcinolon und Triamcinolonderivate, Triamteren, Trifluperidol, Trifluridin, Trimethoprim, Trimipramin, Tripelennamin, Triprolidin, Trifosfamid, Tromantadin, Trometamol, Tropalpin, Troxerutin, Tulobuterol, Tyramin, Tyrothricin, Urapidil, Ursodeoxycholsäure, Chenodeoxycholsäure, Valaciclovir, Valdecoxib, Valproinsäure, Vancomycin, Vardenafil, Vecuroniumchlorid, Venlafaxin, Verapamil, Vidarabin, Vigabatrin, Viloxazin, Vinblastin, Vincamin, Vincristin, Vindesin, Vinorelbin, Vinpocetin, Viquidil, Warfarin, Xantinolnicotinat, Xipamid, Zafirlukast, Zalcitabin, Zanamivir, Zidovudin, Zolmitriptan, Zolpidem, Zoplicon, Zotepin und dergleichen.

Die Wirkstoffe können gewünschtenfalls auch in Form ihrer pharmazeutisch annehmbaren Salze oder Derivate verwendet werden, und im Falle chiraler Wirkstoffe können sowohl optisch aktive Isomere als auch Racemate oder Diastereoisomerengemische eingesetzt werden. Gewünschtenfalls können die erfindungsgemässen Zusammensetzungen auch zwei oder mehrere pharmazeutische Wirkstoffe enthalten.

Beispiele besonders bevorzugter Wirkstoffe sind

Acetylsalicyläure, Carbenoxolon, Cefalotin, Epinefrin, lmipramin, Kaliumjodid, Ketoprofen, Levodopa, Nitrazepam, Nitroprussid, Oxitetracyclin-HCl, Promethazin Omeprazol oder andere Benzimidazolderivate und Streptomycin.

Insbesondere bevorzugt sind feuchteempfindlichen pharmazeutischen Wirkstoffe aus den Wirkstoffklassen der Analgetika, Antirheumatika, Wirkstoffe zur Behandlung von Magengeschwüren, Antibiotika, Antihypotonika, Antidepressiva Schilddrüsentherapeutika, Anti Parkinson-Wirkstoffe, Anxiolytika Peptide, Phospohordiesterasehemmer, Proteine, Herzkreislaufmittel oder Neuroleptika oder deren Salze enthalten ist.

Feuchteempfindliche pharmazeutischer Wirkstoffe sind z. B. Acetylsalicyläure, Carbenoxolon, Cefalotin, Epinefrin, Imipramin, Kaliumjodid, Ketoprofen, Levodopa, Nitrazepam, Nitroprussid, Oxitetracyclin-HCl, Promethazin, Omeprazol oder andere Benzimidazolderivate, Ranitidin oder Streptomycin oder deren Salze.

### Applikationsformen:

Grundsätzlich können die beschriebenen Arzneiformen direkt durch orale Applikation zur Anwendung kommen. Die erfindungsgemäß hergestellten Granulate, Pellets, oder Partikeln können in Gelatinekapseln, Beutel (Sachets) oder in geeignete Mehrdosenbehälter mit Dosiereinrichtung abgefüllt werden. Die Einnahme erfolgt in fester Form oder suspendiert in Flüssigkeiten.
Durch Verpressen erhält man aus, ggf. nach Zumischung weiterer Hilfsstoffe, Tabletten die nach der Einnahme zerfallen und die meist überzogenen Untereinheiten freisetzen. Denkbar ist ebenso die Einbettung von Agglomeraten in Polyethylenglykol oder Lipide zur Herstellung von Suppositorien oder vaginalen Arzneiformen. Überzogene Tabletten werden in Blister oder Mehrdosenbehälter verpackt und vom Patienten direkt vor der Einnahme entnommen.

Wirkstoffklassen und Substanzen, die oftmals bitteren Geschmack hervorrufen können und sich vorteilhaft mit dem erfindungsgemäßen Überzugs- und Bindemittel formulieren lassen sind z. B.:

### Analgetika und Antirheumatika:

Paracetamol, Dictofenac, Aceclofenac, Ibuprofen, Ketoprofen, Flubiprofen, Levacetylmethadol, Oxycodon

### Psychopharmaka:

Prometazine, Donepezil, Modafinil, Nefazodon, Reboxetin, Sertindol, Sertralin

### Antibiotika:

Erythromicyn, Roxithromycin, Clarithromycin, Grepafloxacin, Ciprofloxacin, Levofloxacin, Sparfloxacin, Trovafloxacin, Nevirapin

### Betablocker

Propanolol, Metoprolol, Bisoprolol, Nebivolol

### Antidiabetika:

Metformin, Miglitol, Repaglinid

### H1 Antihistaminika:

Diphenhydramin, Fexofenadin, Mizolastin

### H2 Antihistaminika

Cimetidin, Nizatidin, Ticlopidin, Cetridin, Ranitidin,

Vitamine: Thiaminenitrate; Weitere: Chinidin-Sulfat, Amiloprilose-HCl, Pseudoephedrin-HCl, Sildenafil, Topiramat, Granisetron, Rebamipide, Chinin-HCl

### BEISPIELE

EUDRAGIT^{®} E 100: Copolymer aus Methylmethacrylat, Butylmethacrylat, und Dimethylaminoethylmethacrylat in Verhältnis 25 : 25:
T = Gew.-Teile = Gew.-%

### Beispiel 1:

Herstellung eines farblosen Granulates, welches sich rasch dispergieren lässt.

15 Teile (T) Stearinsäure werden mit 7 T Natriumlaurylsulfat in einem geeigneten Mischer gemischt und anschließend mit 100 T EUDRAGIT^{®} E 100 in einem gleichläufigen 18 mm Doppelschneckenextruder, dessen Schnecken mit Compoundierelementen versehen sind, bei Temperaturen zwischen 105 bis 140 °C mit einem Durchsatz von ca. 1,6 kg/h extrudiert. Der Strang wird über ein Abzugsband abgezogen und einem Stranggranulator zugeführt, welcher den Strang in ca. 3.05 mm (0,12 inch) lange Stücke zerschneidet. Der mittlere Durchmesser der erhaltenen Granulatkörner liegt bei 2 mm und ist abhängig von der Abzugsgeschwindigkeit des Stranges.
15 T des so gewonnenen Granulates lassen sich bei 63 °C mittels eines Magnetrührers in 100 T gereinigtes-Wasser innerhalb 60 min dispergieren. Der aus dieser Dispersion gegossene Film ist homogen.

### Beispiel 2:

### Herstellung eines gelben Granulates, welches sich rasch dispergieren lässt.

15 T Stearinsäure werden mit 10 T Natriumlaurylsulfat und 15 T SICOVIT-Gelb 10 (E 172) in einem geeigneten Mischer gemischt und anschließend mit 100 T EUDRAGIT^{®} E 100 in einem gleichläufigen 18 mm Doppelschneckenextruder, dessen Schnecken mit Compoundierelementen versehen sind, bei Temperaturen zwischen 105 bis 140 °C mit einem Durchsatz von ca. 1,6 kg/h extrudiert. Der Strang wird über ein Abzugsband abgezogen und einem Stranggranulator zugeführt, welcher den Strang in ca. 3.05 mm (0,12 inch) lange Stücke zerschneidet. Der mittlere Durchmesser der erhaltenen Granulatkörner liegt bei 2 mm und ist abhängig von der Abzugsgeschwindigkeit des Stranges.
15 T des so gewonnenen Granulates lassen sich bei 63 °C mittels eines Magnetrührers in 100 T gereinigtes-Wasser innerhalb 60 min dispergieren. Der aus dieser Dispersion gegossene Film ist homogen.

### Beispiel 3:

### Herstellung eines blauen Granulates, welches sich rasch dispergieren lässt.

15 T Stearinsäure werden mit 10 T Natriumlaurylsulfat und 15 T SICOPHARM-Indigotinlack (E 132) in einem geeigneten Mischer gemischt und anschließend mit 100 T EUDRAGIT^{®} E 100 in einem gleichläufigen 18 mm Doppelschneckenextruder, dessen Schnecken mit Compoundierelementen versehen sind, bei Temperaturen zwischen 105 bis 140 °C mit einem Durchsatz von ca. 1,6 kg/h extrudiert. Der Strang wird über ein Abzugsband abgezogen und einem Stranggranulator zugeführt, welcher den Strang in ca. 3.05 mm (0,12 inch) lange Stücke zerschneidet. Der mittlere Durchmesser der erhaltenen Granulatkörner liegt bei 2 mm und ist abhängig von der Abzugsgeschwindigkeit des Stranges.
15 T des so gewonnenen Granulates lassen sich bei 63 °C mittels eines Magnetrührers in 100 T gereinigtes-Wasser innerhalb 60 min dispergieren. Der aus dieser Dispersion gegossene Film ist homogen.

### Beispiele 4:

### Herstellung geschmacksisolierter Chinidinsulfat-Tabletten

Für die Herstellung der Sprühdispersion werden 532 g VE-Wasser auf 63 °C erwärmt vorgelegt. 111,72 g des in Beispiel 2 beschriebenen Granulates werden dem Wasser zugegeben und unter Rühren ca. 60 min dispergiert. Der Dispersion werden als Trennmittel 11,17 g Talkum zugesetzt. Mit der erhaltenen Sprühdispersion werden 3000 g Chinidinsulfat-Keme (⌀10 mm) unter Zuführung von Warmluft besprüht. Die Produkttemperatur liegt zwischen 25 und 33 °C. Anschließend wurden die überzogenen Kerne für zwei Stunden bei 40 °C auf Horden getrocknet. Der erhaltene Filmauftrag ist gleichmäßig (und) deckend und zeigt seine Dichtigkeit durch eine Geschmacksisolierung von größer 30 min.

### Beispiel 5:

### Herstellung einer Dispersion aus zwei verschieden farbigen Granulaten

Je 7,5 T der in Beispiel 2 und 3 beschriebenen Granulate werden in 100 T gereinigtes-Wasser bei 63 °C ca. 60 min dispergiert. Die so erhaltene Dispersion weißt ein homogenes Grün als Farbe auf. Der aus dieser Dispersion gegossene Film überzeugt nach Trocknung ebenfalls mit einem homogenen Farbton.

## Patentansprüche

1. Verfahren zur Herstellung eines Granulats oder Pulvers, geeignet als Überzugs- und Bindemittel für orale oder dermale Arzneiformen, für Kosmetika oder Nahrungsergänzungsmittel, bestehend im wesentlichen aus
(a) einem Copolymer, bestehend aus radikalisch polymerisierten C1- bis C4-Estern der Acryl- oder Methacrylsäure und weiteren (Meth)acrylat-Monomeren die funktionelle tertiäre Aminogruppen aufweisen,
(b) 3 bis 25 Gew.-%, bezogen auf (a), eines Emulgators mit einem HLB-Wert von mindestens 14,
(c) 5 bis 50 Gew.%, bezogen auf (a), einer C₁₂- bis C₁₈-Monocarbonsäure oder einer C₁₂- bis C₁₈-Hydroxylverbindung, die ein Alkanol mit einer endständigen Hydroxylgruppe ist,
wobei die Komponenten (a), (b) und (c) gegebenenfalls unter Zusatz eines pharmazeutischen Wirkstoffs und/oder weiterer üblicher Zuschlagstoffe gleichzeitig oder nacheinander miteinander vermengt oder vermischt werden, in einem heizbaren Mischer geschmolzen, gemischt, die Schmelze abgekühlt und zu einem Granulat oder Pulver zerkleinert wird, wobei die Komponente (a) in Pulverform mit einer mittleren Teilchengröße von über 40 µm oder als Granulat mit einer mittleren Teilchengröße von 0,5 -5 mm eingesetzt wird.

2. Verfahren nach Anspruch 1, **dadurch gekennzeichnet, daß** man als heizbaren Mischer einen Extruder einsetzt.

3. Verfahren nach Anspruch 2, **dadurch gekennzeichnet, daß** man einen Doppelschneckenextruder einsetzt.

4. Verfahren nach einem oder mehreren der Ansprüche 1 bis 3, **dadurch gekennzeichnet, daß** man bei Temperaturen im Bereich von 80 bis 160 °C extrudiert.

## Claims

1. Process for the production of granules or powders, suitable as coating agents and binders for oral or dermal pharmaceutical forms, for cosmetics or food supplements, consisting essentially of
(a) copolymer, consisting of free radical-polymerized C1- to C4-esters of acrylic or methacrylic acid and further (meth)acrylate monomers which contain functional tertiary amino groups,
(b) 3 to 25% by weight, based on (a), of an emulsifier having an HLB of at least 14,
(c) 5 to 50% by weight, based on (a), of a C₁₂- to C₁₈-monocarboxylic acid or of a C₁₂- to C₁₈-hydroxyl compound which is an alkanol having a terminal hydroxyl group,
where the components (a), (b) and (c) are simultaneously or successively blended or mixed with one another, optionally with addition of a pharmaceutical active compound and/or further customary additives, fused in a heatable mixer, mixed, the melt is cooled and comminuted to give granules or powders, where the component (a) is used in powder form with a mean particle size of above 40 µm or as granules with a mean particle size of 0.5 - 5 mm.

2. Process according to Claim 1, **characterized in that** the heatable mixer employed is an extruder.

3. Process according to Claim 2, **characterized in that** a double screw extruder is employed.

4. Process according to one or more of Claims 1 to 3, **characterized in that** extrusion is carried out at temperatures in the range from 80 to 160°C.

## Revendications

1. Procédé pour la fabrication d'un produit granulé ou d'une poudre, appropriés en tant que liant et agent d'enrobage pour des formes pharmaceutiques orales ou dermiques, pour des cosmétiques ou des compléments alimentaires, consistant essentiellement en
(a) un copolymère constitué d'esters en C₁-C₄ de l'acide acrylique ou méthacrylique et d'autres monomères (méth)acrylate qui comportent des groupes amino tertiaires fonctionnels, polymérisables par voie radicalaire ;
(b) 3 à 25 % en poids, par rapport à (a), d'un émulsifiant ayant un indice HLB (équilibre hydrophile-lipophile) d'au moins 14,
c) 5 à 50 % en poids, par rapport à (a) d'un acide monocarboxylique en C₁₂-C₁₈ ou d'un composé hydroxylé en C₁₂-C₁₈ qui est un alcanol comportant un groupe hydroxy en bout de chaîne,
dans lequel les composants (a), (b) et (c) sont simultanément réunis ou mélangés entre eux éventuellement avec addition d'une substance active pharmaceutique et/ou d'autres additifs usuels, fondus dans un mélangeur pouvant être chauffé, mélangés, on refroidit la masse fondue et on la fragmente en un produit granulé ou une poudre, le composant(a) étant utilisé sous forme d'une poudre ayant une taille moyenne de particule de plus de 40 µm ou d'un produit granulé ayant une taille moyenne de particule de 0,5-5 mm.

2. Procédé selon la revendication 1, **caractérisé en ce qu'**en tant que mélangeur pouvant être chauffé, on utilise une extrudeuse.

3. Procédé selon la revendication 2, **caractérisé en ce qu'**on utilise une extrudeuse double vis.

4. Procédé selon une ou plusieurs des revendications 1 à 3, **caractérisé en ce qu'**on effectue l'extrusion à des températures dans la plage de 80 à 160 °C.
